# EUROPEAN PATENT SPECIFICATION

(11) **EP 1 216 416 B1**
(45) Date of publication and mention of the grant of the patent: **15.02.2006**
(21) Application number: 99949102.0
(22) Date of filing: 27.09.1999
(51) Int. Cl.: G01N 33/487

(54) **TEST DEVICE**
PRÜFVORRICHTUNG
DISPOSITIF D'ANALYSE

(43) Date of publication of application: 26.06.2002
(73) Proprietor: HYPOGUARD LIMITED, Woodbridge Suffolk IP12 1PE (GB)
(72) Inventor: Black, Murdo, M., Ipswich Suffolk IP6 9DT (GB); Alton, Robin, Harlow Essex CM17 0JU (GB)
(74) Representative: Gemmell, Peter Alan
(86) International application number: PCT/GB1999/003004
(87) International publication number: WO 2001/023885

(56) References cited:
- EP-A- 0 373 413
- EP-A- 0 732 590
- US-A- 5 395 504
- US-A- 5 407 554
- US-A- 5 525 297
- US-A- 5 797 693

## Description

### BACKGROUND OF THE INVENTION

### 1. Field of the Invention

The present invention relates to a test device for measuring the concentration of an analyte in a fluid sample, notably to a test device for analysing blood glucose or other analytes in bodily fluids.

### 2. Description of the Prior Art

Diabetics regularly need to test samples of their blood to determine the level of blood glucose. The results of such tests may be used to determine levels of medication needed to treat the diabetes at the time. In one known type of system, disposable sensors are used to test the blood. The sensors typically take the form of test strips which are provided with a reagent material that will react with blood glucose to produce an electrical signal. Conductive tracks on the test strip relay the electrical signal to a meter which displays the result. After a sample of blood has been applied to the test strip and the measurement has been taken, the test strip is disposed of. In order to couple the conductive tracks on a test strip with the meter, the test strip needs to be inserted into a sensor holder prior to the start of testing. The sensor holder has corresponding mating electrodes which are brought into electrical contact with the conductive tracks of the test strip. Test devices are known in which a plurality of test strip are provided on a cartridge disc. Each strip is housed in its own sensor slot, and means are provided to eject a test strip from its slot when required, and to automatically locate it in a sensor holder. Examples of test devices with test strip dispensers are described in US Patent No. 5,660,791, European Patent Application No. 0 732 590, and European Patent Application No. 0 738 666. The dispensing devices are relatively complex in construction.

International Patent Application No. WO 98/19159 describes a test device which includes a set of test strips and calibration means corresponding to the test strips. The device includes a docking portion which has a sensor holder for engaging a test strip when a reading is to be taken, and the calibration means removes the need for the user to carry out manual calibration. The device does not automatically locate the test strip in the docking portion, which job is carried out by the user.

It is known from International Patent Application No. WO 99/05966, to provide a test device in which a single test member is re-usable and permanently secured to at least a part of the meter. It is necessary to clean the test member after use before it can be reliably reused.

US 5,395,504 discloses a small sensor for an electrochemical measuring system composed of a measuring apparatus having an electronic circuit, a connecting device, a positioning and advancing device and an eliminating device. The apparatus is adapted to receive the sensor, which has a plurality of active, successively disposable measuring zones. The sensor has applications in the quantitative analysis of glucose in the blood.

EP 0 373 413 discloses an apparatus for measuring blood sugar. A disk-shaped thin plate or long-sized thin plate having a roll appearance is housed in a casing in which openings for penetration of test substances are formed and on which diffusion-limiting membranes are adhered to cover each opening. A drive mechanism is provided in the casing for moving the thin plate. The casing is positioned in a test apparatus body having a concentration measuring electrode therein. Multiple measurements of concentration of test substances are carried out by driving the thin plate to position the diffusion-limiting membrane having a test solution deposited thereon to the position available to make contact with the concentration measuring electrode, while keeping the casing in the test apparatus body. As a result, a plurality of measurements of concentration of test substance is said to be carried out easily.

### SUMMARY OF THE INVENTION

According to an aspect of the present invention there is provided a test device as specified in claim 1. Another aspect of the invention provides a cartridge as specified in claim 14. Preferred features are specified in the dependent claims.

By providing the sensors on a reel, with corresponding electrodes of adjacent sensors connected together, application of a fluid sample to any sensor will produce an electrical signal which will be displayed by the meter. The reel may be advanced by a pre-set distance after each sample reading is taken to provide a fresh sensor at a pre-determined test area, and the used sensor may be cleaned or otherwise treated to prevent or reduce its generation of electrical signals.

The device can be simple in construction and does not require the user to clean sensors or to position them in a docking portion.

In a preferred embodiment, separating means are provided to separate a used sensor from one end of the reel before a subsequent measurement is taken. Separation may be achieved by any suitable means, for example by cutting, tearing, punching, or a combination of these means. For convenience hereinafter, the invention will be described with reference to the use of cutting means to cut a used test strip off from the end of the reel.

Any suitable means may be used to advance the reel, for example a sprocket drive or a friction drive. The reel may simultaneously advanced and cut, or the advance of the reel and the cutting of the reel may take place at different times. In a particularly preferred embodiment, the test area at which a sample of fluid is to be applied to a sensor is in a housing which has a lid. Opening or closing of the lid causes the reel to advance to locate a fresh sensor in the test area. It is preferred that closing of the lid causes indexing of the reel and also causes the end of the reel which carries a used sensor to be cut off.

Used sensors which are cut off from the reel may be discarded. Alternatively, a space may be provided in the housing for receiving and retaining sensors which have been cut from the reel. The cut sensors may be permanently stored in the housing or they may be emptied out from time to time. The area where cut sensors are stored in the housing may optionally be provided with an anti-bacterial agent to reduce odours. To facilitate hygienic disposal of used sensors, a removable container may be provided in the housing to receive cut sensors. The removable container may be disposed of and replaced by a new removable container, or it may be emptied, cleaned and replaced in the housing. Used sensors may be placed in the housing by hand, or they may be placed in the housing automatically.

For simplicity, it is preferred that the contacts of the meter are permanently in contact with the conductive tracks on the reel, and hence with the electrodes of the sensor in the test area. However, it would be possible for the meter contacts to be movable away from contact with the conductive tracks when a reading is not being taken. Because the contacts of the meter are always in contact with the conductive tracks when a reading is being taken, there is no need to locate a sensor in a sensor holder. Electrical connections may be permanently made to the tail of the reel, or sliding contacts may be used at any location before the test area.

The reel may be wound in a coil or drum, or it may be in a serpentine configuration wherein the reel alternately loops in one direction and then in an opposite direction. The serpentine configuration may have the benefit of reducing memory effects in the substrate of the test strips.

The reel may be formed from any suitable material, for example polyester, polyamide, PES, PEEK, PVC or the like. Other suitable materials will be well known to those skilled in the art.

Each sensor may carry all of the electrodes and reagents on one surface. However the reel may optionally be printed on both sides, using printing through holes for electrical connections between the surfaces.

Any convenient number of sensors may be provided on the reel, for example, 50, 75 or 100 sensors.

The test device may be disposed of after the sensors on the reel have been used up. However, it is preferred that the reel (or the remains of the reel) is removable and replaceable, so that the test device may be reused.

### BRIEF DESCRIPTION OF THE DRAWINGS

The invention will now be further described, by way of example, with reference to the following drawings in which:
Figure 1 is a perspective view of a test device in accordance with the present invention, with the lid closed;
Figure 2 is a perspective view of the test device of Figure 1 with the lid open;
Figure 3 is a part vertical-section view of the test device of Figure 1;
Figure 4 is sectional view through part of the device of Figure 1, with the lid open;
Figure 5 is a sectional view corresponding to Figure 4, with the lid closed;
Figure 6 is a part sectional view of part of an alternative embodiment of a test device in accordance with the invention, showing the sensor cutter in a non-cutting position;
Figure 7 is a part sectional view corresponding to that of Figure 6, of another alternative embodiment of a test device in accordance with the invention;
Figure 7a is a sectional view of part of the device of Figure 7, showing an optional arrangement of the reel;
Figure 8 is a plan view of part of a reel suitable for use in a test device in accordance with the invention;
Figure 9 is a plan view similar to Figure 8 of an alternative embodiment of the reel;
Figure 10 is a plan view similar to Figure 8 of a further alternative embodiment of the reel;
Figures 11 to 13 are schematic representations of alternative embodiments of test devices in accordance with the invention; and
Figure 14 is a partial sectional view of a further alternative embodiment of the present invention, showing an arrangement for cutting and storing strips from a reel.

### DETAILED DESCRIPTION

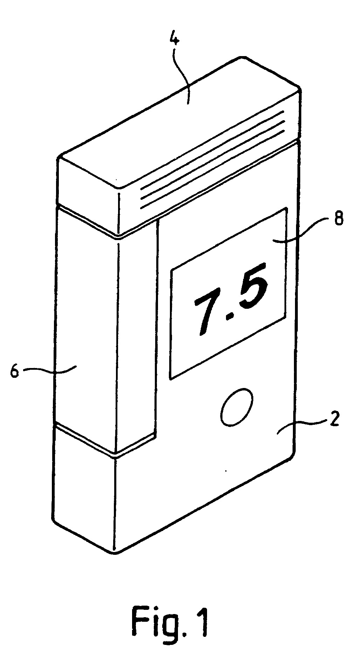

The test device shown in Figure 1 comprises a housing 2 which has a lid 4 connected thereto by a hinge. The housing 2 has a display 8 for displaying an output of a test reading. Without limiting the invention in any way, the dimensions of the housing illustrated are about 90 mm by 50 mm by 15 mm.

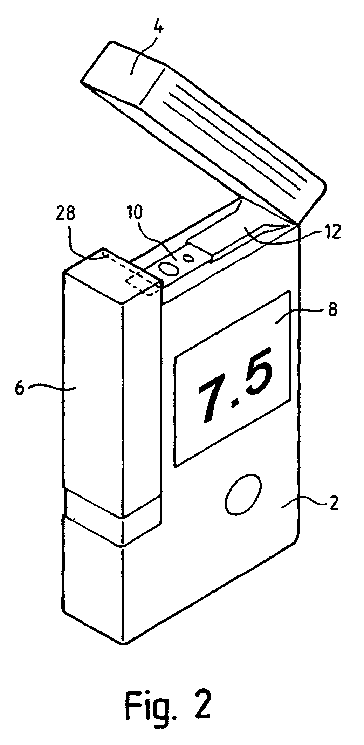

A sensor cutting member 6 is mounted on the housing 2. The sensor cutting member 6 is urged upwards by spring means (not shown) to an extended position as shown in Figure 2. When the lid 4 is closed it pushes the sensor cutting member 6 downwards to a retracted position as shown in Figure 1. The sensor cutting member 6 is provided with a blade 28 along its top inside edge which severs any sensor 10 which is disposed beneath the blade 28 when the lid 4 is closed.

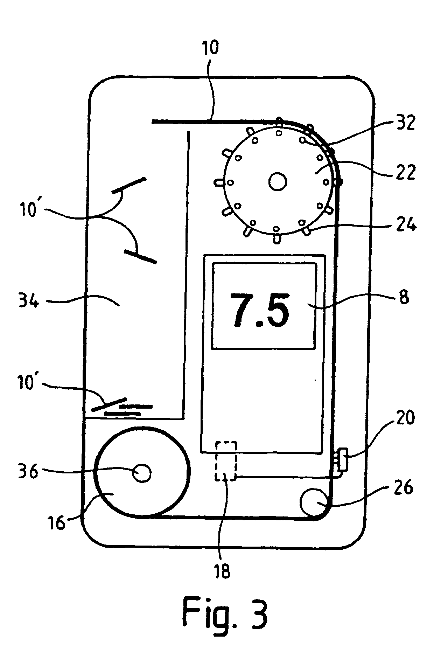

A sensor (test strip) 10, part of a reel 16 as shown in Figure 3, is disposed through a guide member 12 and is exposed in a test area to permit a blood sample to be applied to it. As will be explained presently, closing of the lid 4 causes the reel 16 to advance so that a used sensor 10' is disposed under the blade 28 for cutting and a fresh sensor 10 is exposed in the test area. Cut sensors 10' are collected in a container 34 in the housing 2.

In this example, the reel 16 is made from 100 to 125 µm thick polyester tape. As shown in Figure 3 the reel 16 follows a path within the housing 2 from a spool 36 via a guide wheel 26 and ratchet wheel 22 to the test area at the top of the housing before the blade 28. Other arrangements and paths could of course be used and are within the scope of the invention. Sprocket holes 40 in the tape are engaged by sprockets 24 on the ratchet wheel 22 so that turning of the wheel 22 in a counter-clockwise direction as viewed in Figure 3 advances the reel 16. Sprocket holes could of course alternatively, or additionally, be provided along each edge of the tape in a well-known manner.

The reel 16 could be provided as a removable cartridge which is loaded in the housing in the manner of loading a film in a camera, and it is within the scope of the invention to provide a motorised winding mechanism in the housing 2 for indexing the reel through the housing.

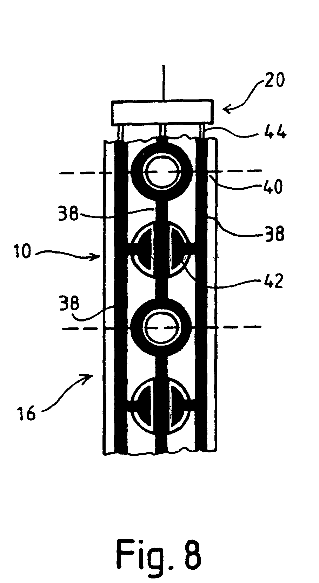

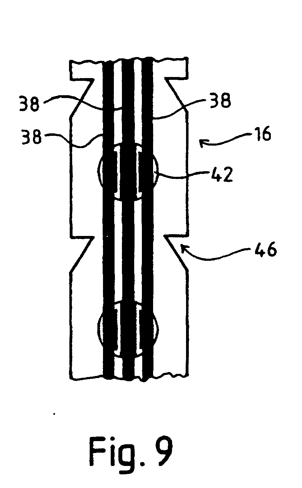

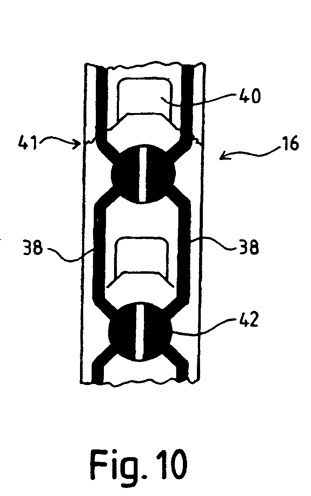

Electronic signal processing means 18 are maintained in electrical contact with outer conductive tracks 38 on the reel 16 by means of contacts 44 in a connector 20, as best shown in Figure 8. Spring biasing means may be employed to help keep the contacts 44 permanently in contact with the reel 16. The reel 16 in Figure 8 has sprocket holes 40 around which the central conductive track is locally disposed. With this arrangement, the central contact 44 will be in electrical contact with the central conductive track only intermittently, when the central contact does not overlie a sprocket hole 40. It is therefore preferred that the path length between the test area and the contacts 44 is selected so that all three contacts 44 are in contact with all three conductive tracks 38 when a test strip 10 is ready for use in the test area. This limitation may be avoided by the use of edge sprockets instead of central sprockets, or notches 46 in the edge of the reel 16 as shown in Figure 9. The notches 46 are engaged by suitable sprockets and provide points of weakness where the reel may be cut or torn. Figure 10 illustrates a two-electrode reel 16 with non-circular sprocket holes 40. This system does not, of course, require a third (central) contact. Lines of weakness 41 are provided in the reel to facilitate tearing off of used sensors.

The signal processing electronics 18 are of course also connected to the display 8 for displaying an output which corresponds to the concentration of analyte (for example glucose) in a fluid sample (for example blood) applied to a sensor 10 at the test area. The signal processing means 18 and the display 8 together comprise the meter which produces a signal output which is dependent on the electrical signal from the sensors 10.

Each sensor 10 (an example of which is shown in Figure 8 between broken lines) comprises a pair of electrodes 42, one of which functions as a working electrode and the other of which is a dummy electrode. A central conductive track 38 functions as a reference/counter electrode.

The reel 16 has 100 sensors 10, all of which sensors are connected together by means of the conductive tracks 38, so that application of an analyte in a fluid sample to any sensor 10 on the reel 16 will produce an electrical signal which is sensed by the signal processing means 18. Since fluid samples are applied only at the test area, which is a fixed distance from the point of contact of the conductive tracks 38 with the contacts 44, the signal processing means may readily be calibrated to produce a display output which corresponds to the concentration of analyte in an applied sample, taking into account factors such as the resistance of the tracks between the two points.

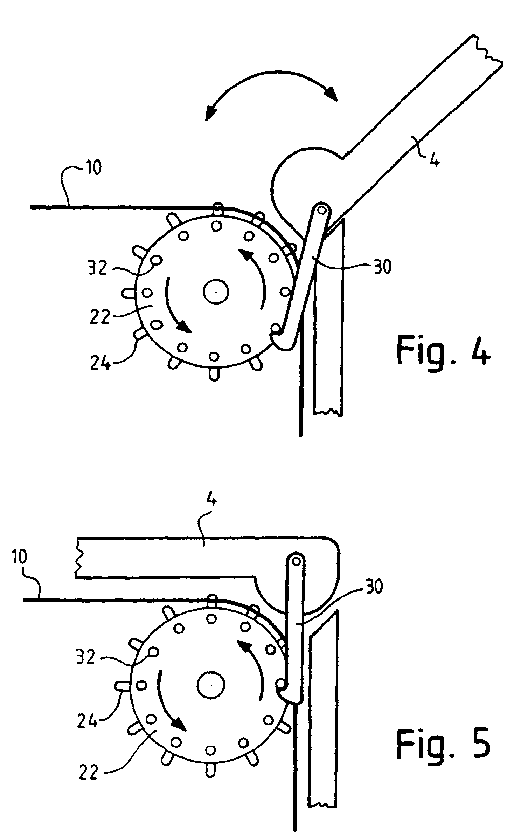

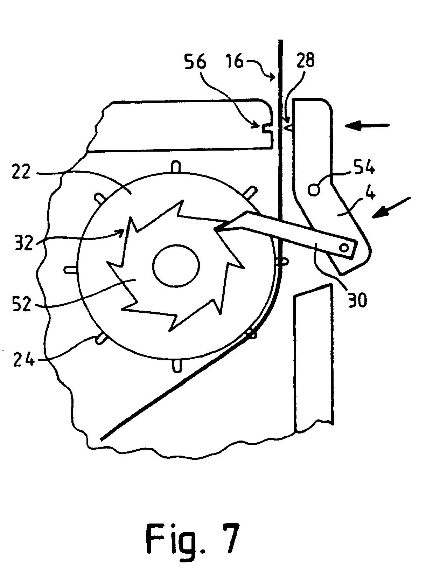

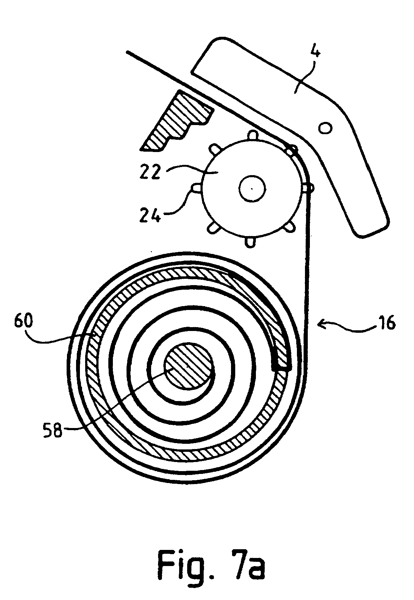

Referring now to Figures 4 and 5, a mechanism for indexing the reel 16 forward is illustrated. The ratchet wheel 22 has a plurality of ratchet teeth 32 radially disposed about one face. The hinged lid 4 is provided with a pawl 30 which is pivotally attached such that the act of closing the lid 4 causes the head of the pawl 30 to engage with and move a ratchet tooth 32, thereby causing the wheel 22 to move counter-clockwise as viewed in the drawings and advance the reel 16 by a distance corresponding to one sensor 10. Fully closing the lid 4 then causes the cutting blade 28 on the sensor cutter 6 to cut off a used sensor 10' as previously described. When the lid 4 is opened, the pawl 30 drops back to the position shown in Figure 7, without moving the wheel 22, and the indexing process can then be repeated.

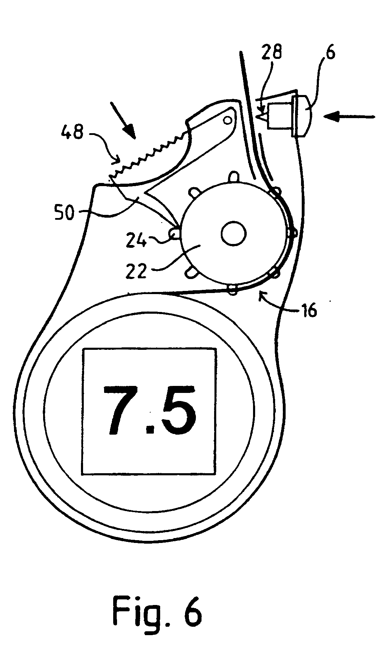

In the embodiment shown in Figure 6, separate mechanisms are provided for advancing and for cutting the reel 16. Sprockets 24 are pushed counter-clockwise as shown in Figure 6 by a pushing member 50 which projects inwardly from a trigger 48 which is pivotally mounted in the housing. Pressure from a user's finger on the trigger 48 depresses the trigger and indexes the reel 16 forward by a distance equal to the length of one sensor 10. The trigger is biased by a spring (not shown) to return the trigger to a rest position at which it may again be depressed to index the reel by the same distance. After indexing the reel 16 once, and taking a sample reading, the user can cut the used sensor 10 from the reel 16 by pressing on a spring-biased sensor cutting member 6 so that a blade 28 cuts the reel.

Figure 7 shows another embodiment, in which the sprocket wheel 22 is provided with a drive wheel 52 whereby turning the drive wheel turns the sprocket wheel. A pawl 30 is pivotally connected to the lower portion of a lid 4 which has a central pivot 54. When a user fully depresses the lower part of the lid 4, the pawl 30 pushes a ratchet tooth 32 on the drive wheel 52 to index the reel by the length of one sensor. After taking a sample reading, the user pushes the upper part of the lid 4 so that a blade 28 cuts the used sensor from the reel 16. A notch or die 56 is provided in a surface under the reel 16 and co-operates with the blade 28 to aid cutting or tearing of the sensor 10.

Figure 7a shows an optional arrangement for storing the reel 16 on a rotatable drum 60. The tail of the reel 16 is fixed to a core 58. Electrical contacts (not shown) on the core 58 are permanently connected to the conductive tracks on the reel 16. As the reel is advanced by the sprocket drive, it unwinds from the outside of the rotatable drum 60. The tail end of the reel unwinds inside the drum, from a small radius around the fixed core to a larger radius, with fewer turns.

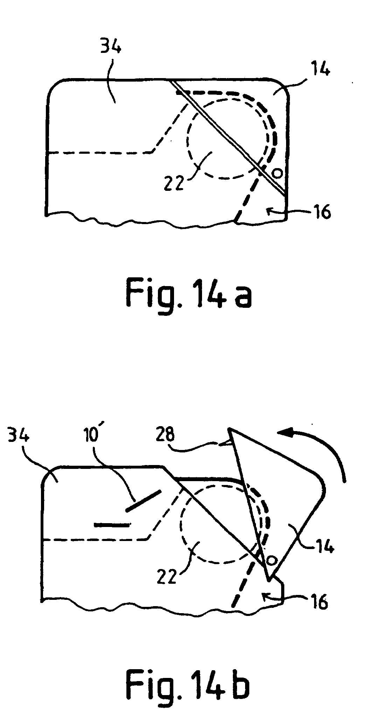

Referring now to Figure 14, another alternative arrangement is shown, similar to the feeding and cutting mechanism shown in Figure 7. Opening the lid 4 (Figure 14a) turns the ratchet wheel 22 and moves a fresh sensor into the test area. Closing the lid (Figure 14a) cuts off the used sensor by means of a blade 28, and the used sensor 10' drops into a waste container 34. The container 34 has one or more antibacterial agents to reduce odours.

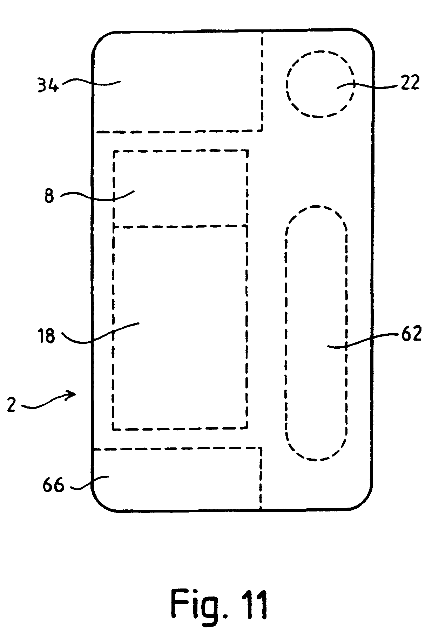

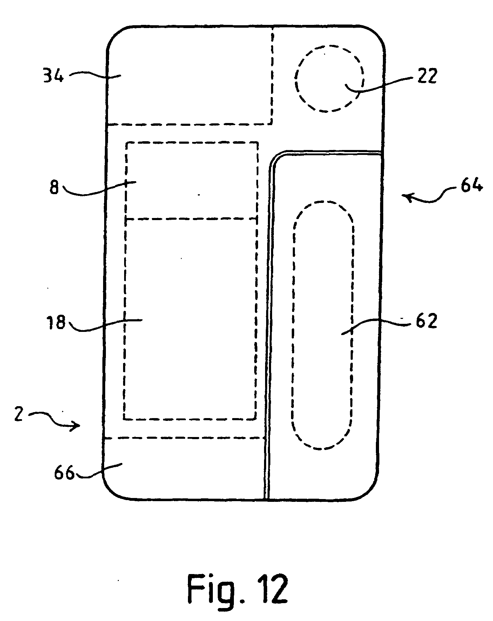

The device shown schematically in Figure 11 is a fully integrated unit which is disposed of when used. The unit comprises a PCB with signal processing electronics 18, a display 8, a battery 66, an optional waste sensor container 34, a reel storage area 62 and a feed mechanism 22 (optionally with a sensor detaching mechanism). The device of Figure 12 has the same component elements, but the reel is stored in a cassette or cartridge 64 which is removable. The reel may be wound on to the feed mechanism 22 automatically by a feed mechanism powered by the battery, or manually, in the manner of a camera wind-on mechanism.

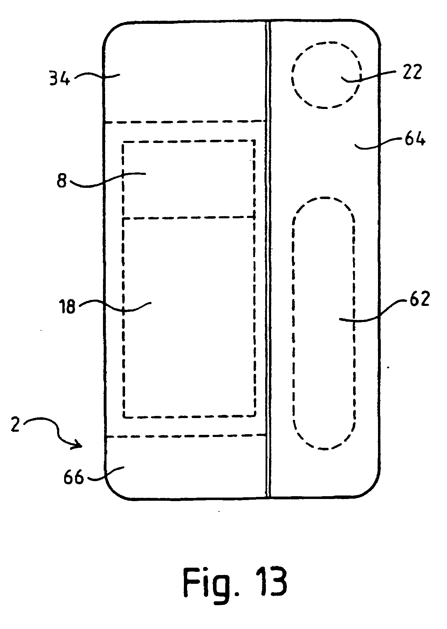

The device shown in Figure 13 features a cassette system in which both the reel storage area 62 and the feed mechanism 22 (and optionally the cutting mechanism) are housed in the cassette 64. Optionally, the cassette 64 could also house the used sensors. The waste sensor container 34 could be provided with a take-up spool on which would be wound the reel 16 after use.

Although the invention has been described with reference to various embodiments, these embodiments are not intended to be limiting. It will be apparent to those skilled in the art that modifications thereto can be made without departure from the scope of the invention as set forth in the following claims.

## Claims

1. A test device for testing of analyte concentration in a fluid to be applied thereto, the device comprising:
a plurality of sensors (10) on a reel (16), each of said sensors (10) carrying reagent means for producing an electrical signal in response to the concentration of analyte in an applied fluid, and each of said sensors (10) having a plurality of electrodes (42), corresponding electrodes (42) of adjacent sensors (10) being connected together by a conductive track (38) on the reel (16); and
a meter comprising electronics means (18) for producing a signal output which is dependent on the electrical signal from the said sensors (10), the meter having contacts (44) which are electrically connected with the said conductive tracks (38), and a test area for application of a sample to the sensors (10); wherein the contacts (44) remain in a fixed location relative to the test area when the reel (16) is advanced.

2. A test device as claimed in claim 1, wherein the meter has contacts (44) which are permanently connected to the said conductive tracks (38).

3. A test device as claimed in claim 1 or claim 2, further including separating means (28) for separating a used sensor (10') from one end of the reel (16).

4. A test device as claimed in claim 3, wherein the separating (28) means comprises cutting means for cutting the reel (16).

5. A test device as claimed in claim 3 or claim 4, wherein a sensor (10) is exposed to permit application of a fluid sample at the test area which is within a housing (2), the housing (2) having a lid (4) which can be moved to cover the test area.

6. A test device as claimed in claim 5, wherein moving the lid (4) from an open position to a closed position causes the reel (16) to advance to locate a fresh sensor (10) in the test area.

7. A test device as claimed in claim 5 or claim 6, wherein closure of the lid (4) causes the separating means (28) to operate to separate a used sensor (10') from one end of the reel (16).

8. A test device as claimed in claim 6, wherein movement of the lid (4) causes the reel (16) to advance by means of a ratchet mechanism (22, 30, 32).

9. A test device as claimed in claim 5, wherein the lid (4) is pivotally mounted in relation to the housing (2), pivoting of the lid (4) in one direction causing the reel (16) to advance so that a fresh sensor (10) is presented in the test area, and pivoting of the lid (4) in another direction causing separation of that sensor from the end of the reel (16).

10. A test device as claimed in any one of the preceding claims, wherein the reel (16) is wound around a rotatable drum (60).

11. A test device as claimed in any one of claims 5 to 10, wherein a container (34) is provided in the housing (2) to receive sensors (10') which have been separated from the reel (16).

12. A test device as claimed in claim 11, wherein the container (34) is removable from the housing (2).

13. A test device as claimed in any one of the preceding claims, wherein the meter is housed in a housing (2) and the reel (16) is provided in a removable cartridge (64) which is mounted in relation to the housing (2).

14. A cartridge (64) for releasably mounting in relation to the housing (2) of a test device in accordance with claim 13, comprising a plurality of sensors (10) on a reel (16), each of said sensors (10) carrying reagent means for producing an electrical signal in response to the concentration of analyte in an applied fluid, and each of said sensors (10) having a plurality of electrodes (42), corresponding electrodes (42) of adjacent sensors (10) being connected together by a conductive track (38) on the reel (16); whereby when the cartridge (64) is mounted in relation to said housing (2) the area where the conductive tracks (38) on the reel (16) touch the contacts (44) will remain at a fixed distance from the test area when the reel (16) is advanced.

15. A cartridge (16) as claimed in claim 14, further including a mechanism for unwinding and advancing the reel when the cartridge (64) is mounted in the housing (2) of a test device.

16. A cartridge (64) as claimed in claim 14 or claim 15, further including storage means (34) for storing used sensors (10').

## Patentansprüche

1. Testvorrichtung zur Bestimmung der Analytkonzentration in einem darauf aufgetragenen Fluid, wobei die Vorrichtung aufweist:
eine Vielzahl von Sensoren (10) auf einem Streifen (16), wobei jeder dieser Sensoren (10) Reagenzmittel zur Erzeugung eines elektrischen Signals in Ansprechen auf die Analytkonzentration in einem aufgetragenen Fluid trägt und jeder dieser Sensoren (10) mit einer Vielzahl von Elektroden (42) ausgestattet ist, wobei entsprechende Elektroden (42) benachbarter Sensoren (10) durch eine Leiterbahn (38) auf dem Streifen (16) miteinander verbunden sind, und
einen Zähler, der elektronische Mittel (18) zur Erzeugung eines Signaloutputs aufweist, der von dem elektrischen Signal von diesen Sensoren (10) abhängt, wobei der Zähler über Kontakte (44) verfügt, die elektrisch mit den Leiterbahnen (38) verbunden sind, und eine Testzone zum Auftragen einer Probe auf die Sensoren (10), wobei die Kontakte (44) bezüglich der Testzone an einem fixierten Ort verbleiben, wenn der Streifen (16) vorwärts bewegt wird.

2. Testvorrichtung nach Anspruch 1, bei der der Zähler über Kontakte (44) verfügt, die mit den Leiterbahnen (38) permanent verbunden sind.

3. Testvorrichtung nach Anspruch 1 oder Anspruch 2, die außerdem Abtrennmittel (28) zum Abtrennen eines verwendeten Sensors (10') von einem Ende des Streifens (16) besitzt.

4. Testvorrichtung nach Anspruch 3, bei der das Abtrennmittel (28) Schneidmittel zum Schneiden des Streifens (16) aufweist.

5. Testvorrichtung nach Anspruch 3 oder Anspruch 4, bei der ein Sensor (10) frei liegt, um das Auftragen einer Fluidprobe in der Testzone zu ermöglichen, die sich innerhalb eines Gehäuses (2) befindet, wobei das Gehäuse (2) einen Deckel (4) besitzt, der bewegt werden kann, um die Testzone abzudecken.

6. Testvorrichtung nach Anspruch 5, bei der durch Bewegen des Deckels (4) von einer geöffneten Position zu einer geschlossenen Position der Streifen (16) vorwärts bewegt wird, um einen frischen Sensor (10) in der Testzone zu platzieren.

7. Testvorrichtung nach Anspruch 5 oder Anspruch 6, bei der durch Schließen des Deckels (4) die Abtrennmittel (28) in Betätigung gesetzt werden, um einen benutzten Sensor (10') von einem Ende des Streifens (16) abzutrennen.

8. Testvorrichtung nach Anspruch 6, bei der durch die Bewegung des Deckels (4) der Streifen (16) mit Hilfe eines Klinkenmechanismus (22, 30, 32) vorwärts bewegt wird.

9. Testvorrichtung nach Anspruch 5, bei der der Deckel (4) bezüglich des Gehäuses (2) schwenkbar befestigt ist, wobei das Schwenken des Deckels (4) in eine Richtung bewirkt, dass der Streifen (16) vorwärts bewegt wird, so dass ein frischer Sensor (10) in der Testzone präsentiert wird, und wobei das Schwenken des Deckels (4) in die andere Richtung die Abtrennung dieses Sensors von dem Ende des Streifens (16) bewirkt.

10. Testvorrichtung nach einem der vorhergehenden Ansprüche, bei der der Streifen (16) um eine drehbare Trommel (60) gewickelt ist.

11. Testvorrichtung nach einem der Ansprüche 5 bis 10, bei der in dem Gehäuse (2) ein Behältnis (34) vorgesehen ist, um Sensoren (10') aufzunehmen, die von dem Streifen (16) abgetrennt worden sind.

12. Testvorrichtung nach Anspruch 11, bei der das Behältnis (34) von dem Gehäuse (2) entfernbar ist.

13. Testvorrichtung nach einem der vorhergehenden Ansprüche, bei der der Zähler in einem Gehäuse (2) untergebracht ist und der Streifen (16) in einer entfernbaren Kartusche (64) bereitgestellt wird, die bezüglich des Gehäuses (2) befestigt ist.

14. Kartusche (64) zur lösbaren Befestigung an dem Gehäuse (2) einer Testvorrichtung gemäß Anspruch 13, enthaltend eine Vielzahl von Sensoren (10) auf einem Streifen (16), wobei jeder dieser Sensoren (10) Reagenzmittel zum Erzeugen eines elektrischen Signals in Ansprechen auf die Analytkonzentration in einem aufgetragenen Fluid aufweist und jeder dieser Sensoren (10) über eine Vielzahl von Elektroden (42) verfügt, wobei entsprechende Elektroden (42) benachbarter Sensoren (10) durch eine Leiterbahn (38) auf dem Streifen (16) miteinander verbunden sind, und wobei, wenn die Kartusche (64) an dem Gehäuse (2) befestigt wird, die Zone, in der die Leiterbahnen (38) auf dem Streifen (16) die Kontakte (44) berühren, in einem fixierten Abstand von der Testzone verbleiben, wenn der Streifen (16) vorwärts bewegt wird.

15. Kartusche (16) nach Anspruch 14, die außerdem einen Mechanismus zum Abwickeln und Vorwärtsbewegen des Streifens besitzt, wenn die Kartusche (64) in dem Gehäuse (2) einer Testvorrichtung befestigt ist.

16. Kartusche (64) nach Anspruch 14 oder Anspruch 15, die außerdem Aufbewahrungsmittel (34) zum Aufbewahren benutzter Sensoren (10') aufweist.

## Revendications

1. Dispositif de test pour tester la concentration d'un analyte dans un fluide devant être appliqué à ce dispositif, le dispositif comprenant :
une pluralité de capteurs (10) sur une bobine (16), chacun desdits capteurs (10) portant des moyens réactifs pour produire un signal électrique en réponse à la concentration d'un analyte dans un fluide appliqué, et chacun desdits capteurs (10) possédant une pluralité d'électrodes (42), des électrodes correspondantes (42) de capteurs adjacents (10) étant connectées entre eux par une piste conductrice (38) sur la bobine (16); et
un appareil de mesure comprenant des moyens électroniques (18) pour produire un signal de sortie, qui dépend du signal électrique délivré par lesdits capteurs (10), l'appareil de mesure comprenant des contacts (44), qui sont connectés électriquement auxdites pistes conductrices (38), et une zone de test pour l'application d'un échantillon aux capteurs (10);
dans lequel les contacts (44) restent dans une position fixe par rapport à la zone de test lorsque la bobine (16) avance.

2. Dispositif de test selon la revendication 1, dans lequel l'appareil de mesure comporte des contacts (44) qui sont connectés de façon permanente auxdites pistes conductrices (38).

3. Dispositif de test selon la revendication 1 ou la revendication 2, comprenant en outre des moyens de séparation (28) pour séparer un capteur utilisé (10') d'une extrémité de la bobine (16).

4. Dispositif de test selon la revendication 3, dans lequel les moyens de séparation (28) comprennent des moyens de coupe pour couper la bobine (16).

5. Dispositif de test selon la revendication 3 ou la revendication 4, dans lequel un capteur (10) est exposé de manière à permettre l'application d'un échantillon de fluide dans la zone de test, qui se situe à l'intérieur d'un boîtier (2), le boîtier (2) comportant un couvercle (4) qui peut être déplacé de manière à recouvrir la zone de test.

6. Dispositif de test selon la revendication 5, dans lequel le déplacement du couvercle (4) depuis une position ouverte vers une position fermée fait avancer la bobine (16) de manière à positionner un capteur neuf (10) dans la zone de test.

7. Dispositif de test selon la revendication 5 ou la revendication 6, dans lequel la fermeture du couvercle (4) amène les moyens de séparation (28) à fonctionner de façon à séparer un capteur utilisé (10') d'une extrémité de la bobine (16).

8. Dispositif de test selon la revendication 6, dans lequel le déplacement du couvercle (4) fait avancer la bobine (16) à l'aide d'un mécanisme à cliquet (22,30,32).

9. Dispositif de test selon la revendication 5, dans lequel le couvercle (4) est monté de manière à pouvoir pivoter par rapport au boîtier (2), le pivotement du couvercle (4) dans une direction amenant la bobine (16) à avancer de sorte qu'un capteur neuf (10) est présenté dans la zone de test, et un pivotement du couvercle (4) dans une autre direction entraînant une séparation de ce capteur par rapport à l'extrémité de la bobine (16).

10. Dispositif de test selon l'une quelconque des revendications précédentes, dans lequel la bobine (16) est enroulée autour d'un tambour rotatif (60).

11. Dispositif de test selon l'une quelconque des revendications 5 à 10, dans lequel un conteneur (34) est prévu dans le boîtier (2) pour recevoir des capteurs (10'), qui ont été séparés de la bobine (16).

12. Dispositif de test selon la revendication 11, dans lequel le conteneur (34) peut être retiré du boîtier (2).

13. Dispositif de test selon l'une quelconque des revendications précédentes, dans lequel l'appareil de mesure est logé dans un boîtier (2) et la bobine (16) est prévue dans une cartouche amovible (64) qui est montée par rapport au boîtier (2).

14. Cartouche (64) destinée à être montée de façon amovible par rapport au boîtier (2) d'un dispositif de test selon la revendication 13, comprenant une pluralité de capteurs (10) situés sur une bobine (16), chacun desdits capteurs (10) portant des moyens réactifs pour produire un signal électrique en réponse à la concentration d'un analyte dans un fluide appliqué, et chacun desdits capteurs (10) possédant une pluralité d'électrodes (42), des électrodes correspondantes (42) de capteurs adjacents (10) étant connectées entre elles par une piste conductrice (38) sur la bobine (16); auquel cas lorsque la cartouche (64) est montée par rapport audit boîtier (2), la surface, au niveau de laquelle les pistes conductrices (38) situées sur la bobine (16) touchent les contacts (94), reste à une distance fixée de la zone de test lorsque la bobine (16) avance.

15. Cartouche (16) selon la revendication 14, comprenant en outre un mécanisme pour dérouler et faire avancer la bobine lorsque la cartouche (64) est montée dans le boîtier (2) d'un dispositif de test.

16. Cartouche (64) selon la revendication 14 ou la revendication 15, incluant en outre des moyens de mémoire (34) pour stocker des capteurs utilisés (10').
